# EUROPÄISCHE PATENTANMELDUNG

(11) **EP 0 884 323 A1**
(43) Veröffentlichungstag der Anmeldung: **16.12.1998**
(21) Anmeldenummer: 98108564.0
(22) Anmeldetag: 12.05.1998
(51) Int. Cl.: C07K 1/113

(54) **Renaturierung von Eiweissstoffen mittels fluorierten Reagenzien**

(30) Priorität: 31.05.1997 DE 19722950
(71) Anmelder: HÜLS AKTIENGESELLSCHAFT, 45764 Marl (DE)
(72) Erfinder: Lohr, Frauke, Dr., 45657 Recklinghausen (DE); Bree, Martina, Dr., 10709 Berlin (DE); Motschmann, Hubert, Dr., 12055 Berlin (DE); Pawlik, Andreas, Dr., 38159 Vechelde (DE); Vieira, Euridice, 4810 Guimaraes (PT); Welle, Alexander, 68526 Ladenburg (DE)

(57) **Zusammenfassung**

Die Erfindung betrifft ein Verfahren zur Renaturierung von denaturierten Eiweißkörpern, bei dem man sie mit einem Renaturierungsmittel behandelt, das an vicinalen Kohlenstoffatomen eine Hydroxylgruppe und mindestens ein Fluoratom trägt.

## Beschreibung

Die Erfindung betrifft ein Verfahrung zur Renaturierung von denaturierten Eiweißstoffen durch Behandlung mit einem Renaturierungsmittel.

In pflanzlichen und tierischen Organismen vorkommende Eiweißstoffe verschiedenster Art mit verschiedenartigen Wirkungen auf die Struktur, die Funktion und den Stoffwechsel der Zellen neigen unter verschiedenen Einflüssen dazu, ihre natürliches räumliches Faltungsmuster, die sogenannte Sekundärstruktur, einzubüßen und zu denaturieren. Die Denaturierung schließt einen Zusammenbruch der intramolekularen Wechselwirkung, insbesondere von Wasserstoffbrücken, und damit einen Verlust der α-Helix-Struktur ein, die nahezu alle nativen Eiweißstoffe zumindest in Teilen des Moleküls aufweisen und die als Teil der Sekundärstruktur für die Aktivität des Eiweißstoffes maßgeblich verantwortlich ist.

Einer der Einflüsse, die zur Denaturierung führen, ist Erhitzen auf eine Temperatur, die je nach Eiweißstoff bis zu 150°C betragen kann. Andere derartige Einflüsse sind Änderungen des pH-Wertes unter 3 oder über 9, Zusatz von denaturierenden Reagenzien, wie Harnstoff-, Guanidin- oder Amidlösungen, Einbringen in schäumende Lösungen. Spreitung an Oberflächen, Bestrahlen mit UV- oder Röntgenstrahlen oder Anwendung von Netzmitteln.

In der Technik werden Eiweißstoffe nicht selten unbeabsichtigt, aber zwangsweise denaturiert, weil ihre Umgebung aus anderen Gründen Bedingungen unterworfen wird, unter denen Denaturierung stattfindet. Ein Beispiel hierfür ist die Dampfsterilisierung von Biomaterialien, bei der darauf fixierte Proteine denaturiert werden. Ein anderes Beispiel ist die UV-Bestrahlung von Polymeroberflächen mit darauf adsorbierten oder immobilisierten Eiweißstoffen zur Vorbereitung einer Pfropfung mit Monomeren, die die Oberflächeneigenschaften Modifizieren sollen. Weiterhin können wertvolle Eiweißstoffe, wie Blutproteine, ungewollt denaturiert werden, beispielsweise indem der pH-Wert bei Anbindung und Modifizierung von Oberflächen in den gefährlichen Bereich gerät.

Es wäre daher erwünscht, über ein Verfahren verfügen zu können, nach dem denaturierte Eiweißkörper renaturierbar sind.

Es wurde nun gefunden, daß denaturierte Eiweißkörper zumindest teilweise renaturiert werden, wenn man sie mit einem Renaturierungsmittel behandelt, das an vicinalen Kohlenstoffatomen eine Hydroxylgruppe und mindestens ein Fluoratom trägt.

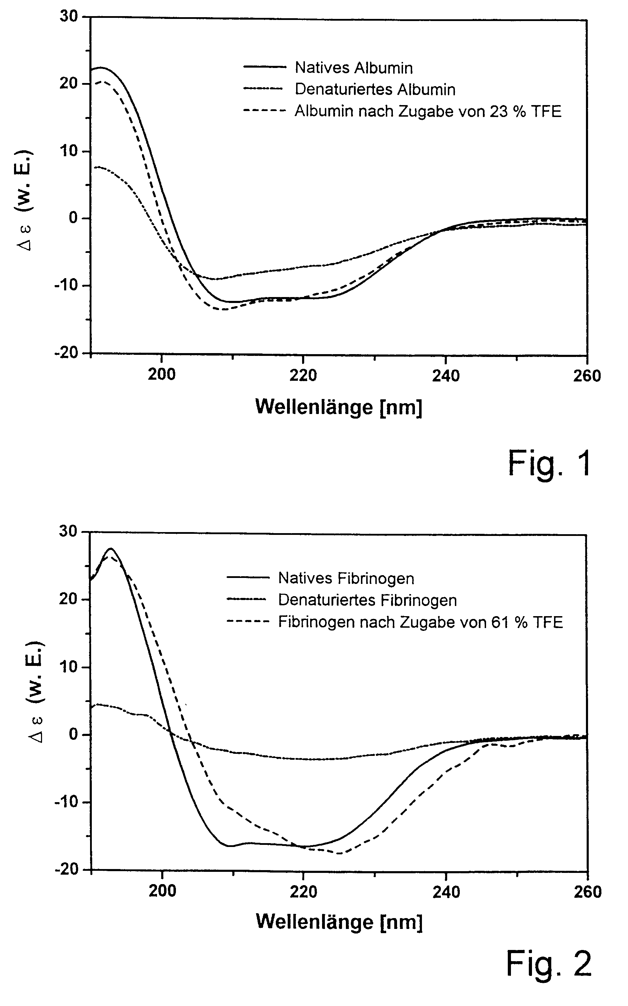

Die Figuren 1 und 2 zeigen die Circulardichroismus(CD)-Spektren von Humanserumalbumin (HSA) und Humanfibrinogen (HFb) im nativen, denaturierten und erfindungsgemäß renaturierten Zustand. Man sieht, daß die Kurven der denaturierten Eiweißstoffe erheblich von den Kurven der ursprünglich nativen Eiweißstoffe abweichen, während die Kurven der renaturierten Eiweißstoffe denen der nativen Eiweißstoffe wieder weitgehend entsprechen.

Es ist zwar bekannt, daß Trifluorethanol die Sekundär- und Tertiärstruktur von Eiweißstoffen konserviert (F.D.Sönnichsen et al., Biochemistry **1992**, 31, 8790-8798; J.S.Albart et al., Biochemistry **1995**, 34, 984-990; A.Cammers-Goodwin et al., J.Am.Chem.Soc. **1996**, 118, 3082-2890) und darüberhinaus nichtspezifische Helixfaltungen induziert, so daß Proteine mit anderen dominanten Faltungsmustern entstehen und hierdurch denaturiert werden (A.J.Arunkumar et al., Biochimica et Biophysica Acta **1997**, 1338, 69-79). Überraschenderweise bilden jedoch denaturierte Eiweißstoffe sogar ihre natürliche Sekundärstruktur wieder aus, so daß ihre biologische Funktion wiederhergestellt wird, wenn sie mit einem der genannten Renaturierungsmittel behandelt werden. Dabei bildet sich die α-Helix zumindest teilweise zurück. Es ist ungeklärt, nach welchem Mechanismus das Renaturierungsmittel bewirkt, daß die erwähnten strukturbestimmenden intramolekularen Wechselwirkungen wieder eintreten, also Wasserstoffbrücken neu geknüpft werden.

Das Ausmaß der erzielbaren Renaturierung ist von Eiweißstoff zu Eiweißstoff verschieden. Unter günstigen Bedingungen wird eine sehr weitgehende Renaturierung erreicht.

Denaturierte Eiweißstoffe im Sinne dieser Erfindung sind solche, die nach der (in der Folge erläuterten) CD-spektroskopischen Analyse ihre ursprüngliche Struktur, bestehend aus α-Helix, β-Faltblatt und anderen Merkmalen, nicht mehr aufweisen. Von den Eiweißstoffen, die nach dem Verfahren der Erfindung renaturiert werden können, seien beispielsweise genannt: Albumine, Fibrinogen, Apolipoproteine, Lysozyme, Subtilisin, Ribonucleasen, Myoglobine, Trypsine, Chymotrypsin, Cytochrom C, Calmodulin, Melittin, Protein C, Globuline, Histone, Prolamine, Protamine, Keratine und Kollagen.

Unter den Renaturierungsmitteln sind diejenigen am wirkungsvollsten. die 3 Fluoratome an einem Kohlenstoffatom enthalten, das einem Kohlenstoffatom mit Hydroxylgruppe benachbart ist, das heißt also eine Trifluormethylgruppe tragen. Die Renaturierungsmittel können, von den erwähnten funktionellen Gruppen abgesehen, Kohlenwasserstoffstruktur besitzen oder zusätzlich Gruppen oder Atome aufweisen, cie den Verwendungszweck nicht stören, wie Carbonestergruppen, Etherbrücken oder Halogenatome. Da die Renaturierung vorteilhaft in wäßrigem Medium stattfindet, sollten die Renaturierungsmittel zumindest in geringem Maße, z.B. zu 1 Gew.-% in Wasser von 20°C, wasserlöslich sein. Gegebenenfalls kann die Wasserlöslichkeit durch zusätzliche hydrophile Gruppen (neben der erwähnten Hydroxylgruppe an einem der vicinalen Kohlenstoffatome) erhöht werden. So können die Renaturierungsmittel beispielsweise weitere Hydroxylgruppen enthalten.

Von den geeigneten Renaturierungsmitteln seien genannt: Monafluorethanol, Difluorethanol und insbesondere Trifluorethanol ; 1-Fluor-2-propanol, 1,1-Difluor-2-propanol 1,1,1-Trifluor-2-propanol, 1,1,1,3-Tetrafluor-2-propanol, 1-Trifluormethyl-1,1-hexandiol und Hexafluorisopropanol.

Das Verfahren der Erfindung wird durchgeführt, indem man das Renaturierungsmittel in einem wäßrigen Medium auf gelöste, suspendierte oder adsorbierte denaturierte Eiweißkörper einwirken läßt, und zwar bei einer Temperatur, die zweckmäßig 0°C bis 60°C beträgt. Die Konzentration des Renaturierungsmittels kann in weiten Grenzen schwanken und z.B. 0,5 bis 95 Vol.-% betragen, bezogen auf das Renaturierungsgemisch, bestehend aus dem wäßrigen Medium mit dem denaturierten Eiweißstoff und dem Renaturierungsmittel. Die Renaturierung nimmt im allgemeinen 5 bis 60 Minuten in Anspruch. Einen Hinweis auf die Renaturierung erhält man durch Circulardichroismus(CD)-Spektroskopie, mit deren Hilfe die absolute Konformation von chiralen (oder optisch aktiven) Verbindungen aufgeklärt werden kann und Konformationsänderungen verfolgt werden können. Hierbei wird linear polarisiertes Licht, im allgemeinen im sichtbaren oder im UV-Bereich, durch die z.B. in Lösung vorliegende oder auf einem durchsichtigen Medium adsorbierte chirale Verbindung gestrahlt. Liegt die eingestrahlte Wellenlänge im Absorptionsbereich der chiralen Verbindung, so können elektronische Übergänge eintreten, die zu einer elliptischen Polarisierung des zuvor linear polarisierten Lichtes führen, das als Überlagerung einer rechts circular polarisierten und einer links circular polarisierten Teilwelle mit gleichen Amplituden aufgefaßt werden kann. Die Absorptionskoeffizienten einer chiralen Verbindung für die rechts circular bzw. links circular polarisierten Teilwellen sind nicht notwendigerweise identisch. Die Differenz der molaren dekadischen Absorptionskoeffizienten für die links circular bzw. die rechts circular polarisierte Teilwelle wird als Circulardichroismus bezeichnet. Nach dem Durchgang durch die Probe überlagern sich die nach der Absorption verbliebenen Anteile der ursprünglichen Teilwellen und ergeben ein elliptisch polarisiertes Licht, da sie im allgemeinen nicht mehr die gleiche Amplitude besitzen. Ändert man die Wellenlänge des eingestrahlten Lichtes, so erhält man ein Spektrum, das den Circulardichroismus als Funktion der Wellenlänge zeigt.

Ebenso kann die Probe nacheinander mit rechts bzw. links polarisiertem Licht durchstrahlt werden, so daß man ein Differenzspektrum gewinnen, das Rückschlüsse auf die Struktur der chiralen Verbindung zuläßt, auch auf deren absolute Konformation.

Zu den mittels CD-Spektroskopie untersuchten chiralen Verbindungen gehören verschiedene Eiweißkörper (oder Proteine). Auch Konformationsveränderungen von Eiweißkörpern an Grenzflächen sind bereits CD-spektroskopisch untersucht worden (siehe z.B. Y.H.Chen et al., Biochemistry, Vol.11, No. 22 (1972), 4120-4131; C.R.MacMillin et al., Journal of Colloid and Interface Science **48**, No.2, (1974) 345-349; L.J.Smith et al., Biochimica et Biophysica Acta, 1121 (1992) 111-118; W.Norde et al., Journal of Colloid and Interface Science, 112, No.2, 447-456; W.Norde et al.. Colloids and Surfaces 62 (1992), 87-93).

Wenn die Eiweißkörper gelöst oder auf Substraten adsorbiert sind. die für Licht der betreffenden Wellenlänge durchlässig sind, kann man Transmissionsmessungen durchführen. Dies ist jedoch nicht möglich bei Adsorption auf lichtundurchlässigen Substraten, für die sich Reflexionsmessungen anbieten. Voraussetzung hierfür ist jedoch, daß eine hinreichend stark reflektierende Oberfläche vorhanden ist, was häufig nicht zutrifft. Ein elegantes Verfahren zur Gewinnung von CD-Spektren adsorbierter Eiweißkörpern (und anderer chiraler Verbindungen) durch Reflexionsmessung ist in der deutschen Patentanmeldung 197 17 431.0 beschrieben.

Zum Hinweis der Renaturierung kann man einen nativen Eiweißkörper mit intakter Sekundärstruktur lösen oder auf einem Substrat adsorbieren und sein CD-Spektrum messen. Dann wird der Eiweißkörper, z.B. durch Erhitzen, denaturiert, und das CD-Spektrum des denaturierten Eiweißkörpers wird gemessen, das von dem des nativen Eiweißkörpers deutlich verschieden ist. Darauf setzt man das Renaturierungsmittel zu. Die Messung des CD-Spektrums ergibt Übereinstimmung mit dem vor der Denaturierung gemessenen Spektrum, wenn ursprüngliche Sekundärstrukturen zurückgebildet worden sind.

Eine Methode zum Nachweis der Renaturierung von Eiweißstoffen und des Ausmaßes der Renaturierung stellt das sogenannte ELISA-Testverfahren (Enzyme Linked Immuno Sorbent Assay) dar. Grundlage dieses Tests ist die spezifische Reaktion von Antikörpern mit Antigenen, die z.B. Bluteiweißstoffe sein können. Die Spezifität bzw. die Bindungsaffinität einer solchen Antigen-Antikörper-Bindung hängt dabei in hohem Maße von der Struktur des Antigens ab. In diesem Zusammenhang spricht man vom Schlüssel-Schloß-Prinzip. Es kommt nur dann zu einer hochaffinen Bindung zwischen Antikörper und Antigen, wenn die molekulare Struktur einer bestimmten Antikörperregion (Schloß) auf eine bestimmte Struktur, z.B. eine Domäne, des Antigens (Schlüssel) abgestimmt ist. Wird im Gegensatz zu nativen Eiweißstoffen die spezifische Bindungsstelle durch Denaturierung zerstört, so ist die Bindung des Antikörpers nicht mehr möglich. Der Nachweis der Renaturierung eines Eiweißstoffes kann also dadurch geführt werden, daß im ELISA-Test eine Reaktion mit dem Antikörper nachweisbar ist, die zuvor nicht stattfand.

Eine Variante des Nachweises ist die Durchführung eines indirekten ELISA-Tests. Hierbei wird in einem ersten Schritt das Antigen an eine Festphase adsorbiert. Im zweiten Schritt wird eine primärer Antikörper an das adsorbierte Antigen gebunden. In einem dritten Schritt wird ein weiterer sogenannter sekundärer Antikörper zugegeben, der spezifische Bindungsstellen für den primären Antikörper besitzet, welcher seinerseits insoweit als Antigen fungiert. Dieser sekundäre Antikörper ist mit einem Enzym konjugiert. In einem vierten Schritt wird der Antigen-Antikörper-Komplex mit einer Substratlösung umgesetzt. Durch Reaktion des angebundenen Enzyms mit dem in der Lösung enthaltenen Substrat wird eine Farbreaktion initiiert, deren optische Dichte proportional der Menge des gebundenen Antigens ist. Die Messung der optischen Dichte und somit der vorhandenen Eiweißstoffkomplexe erfolgt mit einem Spektralphotometer, einem sogenannten Mikrotiterplattenreader. Die für einen solchen Nachweis benötigten entsprechenden Antikörper sind für gängige Eiweißstoffe kommerziell erhältlich. Eine Renaturierung des im ersten Schritt adsorbierten Antigens ist erwiesen, wenn nach der Behandlung mit einem der erfindungsgemäßen Renaturierungsmittel eine Farbreaktion stattfindet, die zuvor ausgeblieben war.

Das erfindungsgemäße Verfahren soll durch die folgenden Beispiele weiter erläutert, nicht aber in seinem Anwendungsbereich beschränkt werden.

### Beispiel 1

Es wurde eine 10⁻⁶ M Lösung von Humanserumalbumin (HSA; Fa. SIGMA) in phosphatgepufferter Lösung (Fa. SIGMA) mit einem pH-Wert von 7,4 hergestellt und das in Figur 1 wiedergegebene CD-Spektrum des nativen Humanserumalbumins gemessen. Dann wurde die Lösung 30 min auf 85°C erwärmt, wodurch der Eiweißstoff denaturiert wurden. Anschließend wurde ein CD-Spektrum des denaturierten Eiweißstoffes in Lösung aufgenommen (ebenfalls Figur 1), dessen Kurvenverlauf sich erheblich von dem des Spektrums des nativen Eiweißstoffes unterscheidet. Dann wurde soviel Trifluorethanol zugesetzt, daß dessen Konzentration im wäßrigen Medium 23 Vol-% betrug. Nach 10 min wurde ein weiteres CD-Spektrum aufgenommen (ebenfalls Figur 1), dessen Kurvenverlauf dem ursprünglichen Spektrum stärker ähnelt als dem des denaturierten HSA, was auf eine Renaturierung hindeutet.

Eine Bestätigung der Regeneration der biologischen Funktionen des Albumins durch die Zugabe von Trifluorethanol wurde mit dem ELISA-Test erhalten. Hierzu wurden Träger mit einer Beschichtung eines methylterminierten Silanfilms (Octyltrichlorsilan der Fa. Aldrich) verwendet. Drei solcher Träger wurden zur Adsorption von Eiweißstoffen 30 min in eine phosphatgepufferte Lösung (Fa. SIGMA) von Humanserumalbumin (Fa. SIGMA) mit einem pH-Wert von 7,4 und einer Konzentration an Eiweißstoffen von 30 µg/ml getaucht. Zwei der mit HSA beschichteten Träger wurden zur Denaturierung der Eiweißstoffe in reinem Phosphatpuffer 30 min auf 85°C erwärmt. Eine dieser Proben wurde anschließend 5 min mit einer 20 %-igen Lösung von Trifluorethanol in Phosphatpuffer nachbehandelt. An allen drei Proben wurden ELISA-Messungen durchgeführt, wofür polyklonale Antikörper verwendet wurden. Die hierbei erhaltenen Signalintensitäten sind in der nachfolgenden Tabelle aufgeführt, wobei auf das Signal der nativen Eiweißstoffe normiert wurde:

| Beschichtung des Trägers | ELISA-Signal [%] |
|---|---|
| adsorbiertes Albumin -> nativ | 100 |
| adsorbiertes Albumin -> denaturiert | 73 |
| adsorbiertes Albumin -> denaturiert und mit Trifluorethanol behandelt | 77 |

### Beispiel 2

Es wurde eine 10⁻⁶ M Lösung von Humanfibrinogen (HFb; Fa. SIGMA) in phosphatgepufferter Lösung (Fa. SIGMA) mit einem pH-Wert von 7,4 hergestellt und das in Figur 2 wiedergegebene CD-Spektrum des nativen HFb gemessen. Dann wurde die Lösung 30 min auf 85°C erwärmt, wodurch der Eiweißstoff denaturiert wurden. Anschließend wurde ein CD-Spektrum des denaturierten Eiweißstoffes in Lösung aufgenommen (ebenfalls Figur 5), dessen Kurvenverlauf sich erheblich von dem des Spektrums des nativen Eiweißstoffes unterschied. Dann wurde soviel Trifluorethanol zugesetzt, daß dessen Konzentration im wäßrigen Medium 61 Vol-% betrug. Nach 10 min wurde ein weiteres CD-Spektrum aufgenommen (ebenfalls Figur 2), dessen Kurvenverlauf dem ursprünglichen Spektrum stärker ähnelte als dem des denaturierten HF, was auf eine Renaturierung hindeutet.

Eine Bestätigung der Regeneration der biologischen Funktionen des Albumins durch die Zugabe von Trifluorethanol wurde mit dem ELISA-Test erhalten. Hierzu wurden Träger mit einer Beschichtung eines methylterminierten Silanfilms (Octyltrichlorsilan der Fa. Aldrich) verwendet. Drei solcher Träger wurden zur Adsorption von Eiweißstoffen 30 min in eine phosphatgepufferte Lösung (Fa. SIGMA) von Humenfibrinogen (Fa. SIGMA) mit einem pH-Wert von 7,4 und einer Konzentration an Eiweißstoffen von 30 µg/ml getaucht. Zwei der mit HFb beschichteten Träger wurden zur Denaturierung der Eiweißstoffe in reinem Phosphatpuffer 30 min auf 85°C erwärmt. Eine dieser Proben wurde anschließend 5 min mit einer 61 %-igen Lösung von Trifluorethanol in Phosphatpuffer nachbehandelt. An allen drei Proben wurden ELISA-Messungen durchgeführt, wofür polyklonale Antikörper verwendet wurden. Die hierbei erhaltenen Signalintensitäten sind in der nachfolgenden Tabelle aufgeführt, wobei auf das Signal der nativen Eiweißstoffe normiert wurde:

| Beschichtung des Trägers | ELISA-Signal [%] |
|---|---|
| adsorbiertes Fibrinogen -> nativ | 100 |
| adsorbiertes Fibrinogen -> denaturiert | 64,4 |
| adsorbiertes Fibrinogen -> denaturiert und mit Trifluorethanol behandelt | 79,2 |

## Patentansprüche

1. Verfahren zur Renaturierung von denaturierten Eiweißkörpern, dadurch gekennzeichnet, daß man sie mit einem Renaturierungsmittel behandelt, das an vicinalen Kohlenstoffatomen eine Hydroxylgruppe und mindestens ein Fluoratom trägt.

2. Verfahren nach Anspruch 1, dadurch gekennzeichnet, daß das Renaturierungsmittel eine Trifluormethylgruppe enthält.

3. Verfahren nach Anspruch 2, dadurch gekennzeichnet, daß das Renaturierungsmittel Trifluorethanol ist.

4. Verfahren nach einem der Ansprüche 1 bis 3, dadurch gekennzeichnet, daß die Renaturierung in einem wäßrigen Medium bei einer Temperatur von 0 bis 60 °C durchgeführt wird.

5. Verfahren nach einem der Ansprüche 1 bis 4, dadurch gekennzeichnet, daß die Konzentration des Renaturierungsmittels im Renaturierungsgemisch 1 bis 95 Vol-% beträgt.
